# EUROPEAN PATENT SPECIFICATION

(11) **EP 2 256 184 B1**
(45) Date of publication and mention of the grant of the patent: **08.04.2015**
(21) Application number: 09717743.0
(22) Date of filing: 04.03.2009
(51) Int. Cl.: C12N 1/18, C12P 19/34, C12R 1/865

(54) **Saccharomyces cerevisiae mutants and a production process of RNA-rich yeast utilizing the mutants**
MUTANTENSTAMM VON SACCHAROMYCES CEREVISIAE UND VERFAHREN ZUR HERSTELLUNG VON HEFE MIT HOHEM RNA-GEHALT MITHILFE DES MUTANTENSTAMMS
SOUCHE MUTANTE DE SACCHAROMYCES CEREVISIAE ET MÉTHODE DE PRODUCTION DE LEVURE COMPRENANT UN TAUX D'ARN ÉLEVÉ UTILISANT LA SOUCHE MUTANTE

(30) Priority: 06.03.2008 JP 2008056840
(43) Date of publication of application: 01.12.2010
(73) Proprietor: Asahi Group Holdings, Ltd., Tokyo 130-8602 (JP)
(72) Inventor: YAMAMOTO, Kazue, Moriya-shi Ibaraki 302-0106 (JP); MATSUMOTO, Takeshi, Moriya-shi Ibaraki 302-0106 (JP)
(74) Representative: Desaix, Anne
(86) International application number: PCT/JP2009/054073
(87) International publication number: WO 2009/110507

(56) References cited:
- JP-A- 5 176 757
- JP-A- 10 117 794
- JP-A- 11 196 859
- JP-A- 53 088 362
- JP-A- 2002 101 846
- JP-A- 2002 272 450
- JP-A- 2006 129 834
- JP-A- 2007 020 430
- JP-B1- 46 010 913
- JP-B2- 38 023 088
- JP-B2- 40 002 869
- KR-A- 20030 018 736
- KIM ET AL.: 'Selection of Yeast Mutant Strain with High RNA Content and Its High Cell-Density Fed-Batch Culture' KOR.J. MICROBIOL, BIOTECHNOL vol. 30, no. L, pages 68 - 72, XP008139318
- KIM ET AL.: 'Effect of Ca++ and Cu++ Removal from Molasses on Yeast cell Growth and RNA Accumulation' KOR.J. MICROBIOL, BIOTECHNOL vol. 31, no. 3, pages 211 - 215, XP008139317

## Description

### TECHNICAL FIELD

The present invention relates to a *Saccharomyces cerevisiae* mutant strain having a high RNA content, a method for producing a yeast strain having a high RNA content using the mutant strain, and a culture and yeast extract of the mutant strain. The present application claims priority on Japanese Patent Application No. 2008-056840, filed March 6, 2008.

### BACKGROUND ART

*Saccharomyces* yeasts such as a brewer's yeast and a baker's yeast contain a good balance of natural vitamin B group, amino acids, minerals, and so on and so forth, and they are effectively used not only for the production of beer and bread but also for other purposes. For example, dry yeast has been used in Japan for many years as a pharmaceutical product, a food ingredient, a flavoring material, or the like, and has been acknowledged as a raw material with a high nutritional value and a high level of safety. In addition, dry yeast has also been widely used as a starting material yeast for producing yeast extracts in recent years.

The yeast extract is prepared from a yeast culture and contains an abundance of amino acids or the like. Accordingly, they have hitherto been used as a food additive such as a flavoring material for providing a flavor or a rich taste. In particular, due to the growing interests for natural products in recent years, the demand for the yeast extracts as a flavoring material has been increasing. Since it is expected that the yeast extracts prepared from the yeast cells containing an abundance of taste components may be used as an even more excellent flavoring material, studies for developing yeast cells containing an even greater amount of taste components have been conducted intensively.

For example, inosinic acid and guanylic acid which are nuclear acidic taste components can be produced by subjecting ribonucleic acid (RNA) to an enzymatic treatment or the like. For this reason, by preparing yeast extracts using the yeast containing a large amount of RNA, it is possible to obtain a yeast extract containing an abundance of nuclear acidic taste components and in which the amount of flavor components is increased.

Various methods and processes have been disclosed in order to obtain a yeast strain having a high RNA content. Examples thereof include: (1) a mutant strain of *Candida utilis* exhibiting cold sensitivity and produces/accumulates 20% by weight or more of RNA based on the yeast cell weight, and a method for producing a yeast strain of high RNA content by aerobically culturing the mutant strain, as a result of which 20% by weight or more of RNA is produced/accumulated in the yeast cells (for example, refer to Patent Document 1); and (2) a newly found yeast strain *Candida tropicalis* having an extremely high RNA content in the cell, and a method for producing yeast RNA characterized by culturing the yeast strain and extracting RNA from the cultured yeast cells (for example, refer to Patent Document 2).
[Patent Document 1] Japanese Unexamined Patent Application, First Publication No. Hei 11-196859
[Patent Document 2] Japanese Patent Application, Second Publication No. Sho 55-49837

### DISCLOSURE OF THE INVENTION

Although the yeasts disclosed in Patent Documents 1 and 2 are both yeasts of high RNA content, they are *Candida* yeasts, and in the *Saccharomyces* yeasts which are generally used for food, in particular, in *Saccharomyces cerevisiae,* such a mutant strain having a high content of RNA has not been known.

Moreover, the yeast strain disclosed in Patent Document 1 is cold sensitive, and thus its growth rate declines when the incubation temperature drops down to 30°C or less. However, in the method disclosed in Patent Document 1, RNA is highly accumulated in the yeast cells by culturing the cells under a culture condition where the growth rate of the yeast cells is somewhat declined. Accordingly, the discrepancy between the optimal conditions for cell growth and the optimal conditions for RNA accumulation has been a problem.

It is also disclosed in either -document KR 2003 0018736 A or in Kim et al ("Selection of Yeast Mutant Strain with High RNA Content and Its High Cell-Density Fed-Batch Culture", KOR. J.MICROBIOL, BIOTECHNOL, vol. 30, no. L, pages 68-72) the *Saccharomyces cerevisiae* mutant strain M40-10, which contains an increased RNA content, said strain being however obtained by mutation of *Saccharomyces cerevisiae* using a chemical mutagen that is ethylmethane sulfonate (EMS).

An object of the present invention is to provide a *Saccharomyces cerevisiae* mutant strain having a high RNA content as well as favorable growth properties, a method for producing a yeast strain having a high RNA content using the mutant strain, and a culture and yeast extract of the mutant strain.

The present inventors have conducted an intensive an extensive study in order to solve the above problems. As a result, a *Saccharomyces cerevisiae* ABYC 1591 strain having a high RNA content as well as favorable growth properties was discovered by screening the yeast library of *Saccharomyces cerevisiae* for yeast strains of high RNA content followed by the back crossing of the yeast strains, and the present invention was completed.

(1)- The present invention relates to *a Saccharomyces cerevisiae* mutant strain, characterized in that the *Saccharomyces cerevisiae* mutant strain is an ABYC 1591 (FERM BP-10925) strain.
(2) The present invention provides a *Saccharomyces cerevisiae* mutant strain characterized by having an RNA content of 14% by weight or more based on the dry cell weight.
(3) The present invention also provides a *Saccharomyces cerevisiae* mutant strain according to the above (1) characterized by having an RNA content of 18% by weight or more based on the dry cell weight.
(4) The present invention also provides a method for producing a yeast of high RNA content, the method characterized by including a step of culturing the *Saccharomyces cerevisiae* mutant strain according to any one of the above (1) to (3), and thereby making the mutant strain accumulate, in the cells thereof, 14% by weight or more of RNA based on the dry cell weight.
(5) The present invention also provides a culture of the *Saccharomyces cerevisiae* mutant strain according to any one of the above (1) to (3).
(6) The present invention also provides a yeast extract prepared from a culture of the *Saccharomyces cerevisiae* mutant strain according to any one of the above (1) to (3).
(7) The present invention also provides a yeast extract according to the above (6) characterized by having an RNA content of 35% by weight or more.
(8) The present invention also provides a yeast extract according to the above (7) **characterized in that** the total content of inosinic acid (that is, inosine monophosphate (IMP)) and guanylic acid (that is, guanosine monophosphate (GMP)) is 20% by weight or more.

### EFFECT OF THE INVENTION

The *Saccharomyces cerevisiae* mutant strain of the present invention is a yeast strain having a high RNA content as well as favorable growth properties. For this reason, by culturing the *Saccharomyces cerevisiae* mutant strain, a yeast of high RNA content can be easily produced. In addition, a culture and a yeast extract of the *Saccharomyces cerevisiae* mutant strain have a sufficiently high RNA content, and thus the strength of flavor is increased, making them highly suitable as flavoring materials.

### BEST MODE FOR CARRYING OUT THE INVENTION

In the present invention, the phrase "dry cell weight" refers to the cell weight which is measured after the cells have been dried. The weight of the cells after a drying process can be determined, for example, by the following procedures. First, the yeast cells are recovered as a pellet by treating a yeast culture through a centrifugal separation process. The recovered cells are washed twice with water through the centrifugal separation process, and thereafter, dried at 105°C for 5 hours, and then the weight of the resultant is measured. In addition, the RNA content of the *Saccharomyces cerevisiae* mutant strain of the present invention based on the dry cell weight can also be determined by the method commonly employed for determining the RNA content in the microbial cells.

The *Saccharomyces cerevisiae* mutant strain of the present invention is characterized by having an RNA content of 14% by weight or more based on the dry cell weight. This is because, by making the RNA content high, that is, 14% by weight or more based on the dry cell weight, it will be possible to easily enhance the RNA content in the culture, yeast extract, or the like obtained from this *Saccharomyces cerevisiae* mutant strain. In the *Saccharomyces cerevisiae* mutant strain of the present invention, it is preferable that the RNA content be 18% by weight or more based on the dry cell weight.

Such a *Saccharomyces cerevisiae* mutant strain can be acquired, for example, through the following procedures.

### 1. Screening for a Saccharomyces cerevisiae mutant strain of high RNA content

First, the present inventors determined the RNA content in about 200 available yeast strains of *Saccharomyces cerevisiae* including those that were commercially available. Specifically, each yeast strain of *Saccharomyces cerevisiae* was inoculated in 10 mL of YPD medium (containing 2% peptone, 1% yeast extract, and 2% glucose) and preincubated overnight in an incubator set at 30°C. The obtained preculture liquid was inoculated to 50 mL of fresh YPD medium so as to achieve the initial cell concentration of 1 × 10⁷ cells/ml, and the cells were shake cultured in an incubator set at 30°C for 16 hours at a rotation speed of 200 rpm.

Cells were recovered from the obtained culture and the RNA content in the cells was measured in the same manner as that in the working Example 1 described later.

As a result, the RNA content in most yeast cells was less than about 10% by weight based on the dry cell weight, and only 5 yeast strains had an RNA content of 10% by weight or more. In addition to these 5 strains, 1 yeast strain which had an RNA content of less than 10% by weight based on the dry cell weight was selected as a control.

The RNA content from the selected 6 yeast strains was measured once again by the same method. The RNA content in each yeast strain obtained as a result of the measurement is shown in Table 1.

**[Table 1]**

| Yeast strain | No. 1 (Control) | No. 2 | No 3 | No. 4 | No. 5 | No. 6 |
|---|---|---|---|---|---|---|
| RNA (% by weight) | 7.61 | 10.94 | 15.08 | 15.98 | 15.83 | 17.76 |

As shown in Table 1, the RNA content in the strain No. 1 was only about 8% by weight based on the dry cell weight whereas the other 5 strains all had an RNA content of 10% by weight or more. Among them, 4 strains from the strain No. 3 to the strain No. 6 all had an RNA content of 14% by weight or more, showing that these strains were yeast strains of high RNA content.

In particular, the strain No. 6 had a considerably high RNA content of about 17.8% by weight.

### 2. Modification of strain No. 6

The strain No. 6 which had the highest RNA content was a haploid yeast strain and had a problem in terms of stability. On the other hand, although a parent strain of the strain No. 6 was a diploid yeast strain, the growth of this strain was extremely poor, and thus it was an unfavorable yeast strain from the viewpoint of industrial application.

Hence, by back crossing the strain No. 6 with the parent strain thereof, a mutant strain of high RNA content is prepared, which has favorable growth properties and which is diploid and is therefore stable.

Specifically, a tetrad analysis was first conducted using the parent strain of the strain No. 6, thereby selecting a spore that has favorable growth properties and also an RNA content of 14% by weight or more based on the dry cell weight. The tetrad analysis was conducted once more after crossing the selected spore with the strain No. 6, and thereby selecting a spore that has favorable growth properties and also an RNA content of 14% by weight or more based on the dry cell weight. This selected spore was picked as a back cross completed strain.

Note that the RNA content in each of the yeast strains was measured in the same manner as that in the working Example 1 described later. In addition, the growth properties of each of the yeast strains were determined by first subjecting the culture medium after the incubation to a centrifugal separation process at 15,000 rpm, and then calculating the cell concentration from the dry weight of the cells, obtained as a pellet due to the separation process, per 100 mL of the culture medium.

**[Table 2]**

| | Parent strain | Back cross completed strain |
|---|---|---|
| Yeast cell concentration | 0.045% | 0.10% |
| RNA content | 15.7% | 15.3% |

Table 2 is a table showing the results of the growth properties as well as the RNA content which are obtained from the parent strain of the strain No. 6 and the back cross completed strain. The growth properties of each strain are presented as the cell concentration after the incubation. From these results, it is apparent that the back cross completed strain was a yeast strain of high RNA content similar to the parent strain of the strain No. 6, and at the same time, it had sufficiently improved growth properties as compared to the parent strain.

Next, a mutant strain was prepared by crossing the obtained back cross completed strain with the strain No. 6. By measuring the growth properties and the RNA content of the obtained mutant strain, a mutant strain having favorable growth properties and also an RNA content of 14% by weight or more based on the dry cell weight is selected. As a result, a yeast strain of the present invention which has a high RNA content as well as excellent growth properties was obtained. The obtained yeast strain was designated as ABYC 1591 strain.

### 3. Mycological properties of ABYC 1591 strain

The ABYC 1591 strain has the same mycological properties as those of the common *Saccharomyces cerevisiae* apart from its RNA content of 14% by weight or more based on the dry cell weight.

When the ABYC 1591 strain is grown on an YPD plate medium containing 2% agar at 30°C for 2 days, the obtained colonies have the following morphological characteristics.
(1) Size: diameter of about 2 mm; (2) Color tone: white to cream colored; (3) Shape: circular shape in which the entire margin thereof is elevated in a half lens form; (4) Surface profile: smooth; (5) Transparency: opaque; and (6) Viscosity: butter-like.

The ABYC 1591 strain obtained in this manner described so far is a mutant strain of *Saccharomyces cerevisiae* having an RNA content of 14% by weight or more based on the dry cell weight, which is the mutant strain of *Saccharomyces cerevisiae* according to the present invention.

In addition, the ABYC 1591 strain is a mutant strain of *Saccharomyces cerevisiae* newly prepared in the present invention by crossing the spore obtained as a result of the tetrad analysis with the IFO 10611 strain, and thus the ABYC 1591 strain is a yeast strain having a much higher RNA content compared to the known strains as well as favorable growth properties, which is a feature conventionally unavailable. Accordingly, the Applicants deposited the ABYC 1591 strain at the International Patent Organism Depositary in the National Institute of Advanced Industrial Science and Technology, Central 6 (1-1-1 Higashi Tsukuba, Ibaraki 305-8566, Japan) as a new yeast strain. The deposition number is FERM BP-10925 and the deposition date is October 19, 2007.

By culturing the *Saccharomyces cerevisiae* mutant strain of the present invention and thereby making the mutant strain to accumulate, in the cells thereof, 14% by weight or more of RNA based on the dry cell weight, the yeast of high RNA content can be produced simply and easily. The culture medium used for culturing the *Saccharomyces cerevisiae* mutant strain of the present invention contains a carbon source, a nitrogen source, inorganic salt, and the like, and it is not particularly limited as long as it is a culture medium in which the *Saccharomyces cerevisiae* mutant strain can proliferate, and any culture medium commonly used for culturing yeasts such as *Saccharomyces cerevisiae* can be used.

As the carbon source contained in a culture medium for culturing the *Saccharomyces cerevisiae* mutant strain of the present invention, for example, at least one carbon source selected from the group consisting of glucose, sucrose, acetic acid, ethanol, molasses, sulfite waste liquor, and the like, which are commonly used for culturing microbes, can be used. In addition, the nitrogen source may be an inorganic salt containing nitrogen or may be organic matter containing nitrogen. For example, at least one nitrogen source selected from the group consisting of urea, ammonia, ammonium sulfate, ammonium chloride, ammonium phosphate, corn steep liquor (CSL), casein, yeast extracts, peptone, and the like can be used. In addition, as an inorganic salt, a phosphoric acid component such as superphosphate of lime and ammonium phosphate, a potassium component such as potassium chloride and potassium hydroxide, a magnesium component such as magnesium sulfate and magnesium chloride, and the like can be used. Other inorganic salts of zinc, copper, manganese, iron ions, and the like may also be used. Moreover, in a culture medium for culturing the *Saccharomyces cerevisiae* mutant strain of the present invention, vitamins, nucleic acid related materials, or the like may also be added where appropriate. Examples of such culture media include the YPD medium.

There is no particular limitation on the culturing mode and it can be determined appropriately by taking the culture scale, application of the obtained culture, and the like into account. For example, the yeast cells may be streaked and cultured on an agar plate medium or may be cultured in a liquid culture medium. Examples of the culture mode when using a liquid culture medium include batch cultivation, fed batch cultivation, and continuous cultivation. For example, when subculturing yeast cells, it is preferable to culture cells on an agar plate medium or in an adequate liquid culture medium as a batch culture, due to the simplicity of the process. Also, when producing a yeast of high RNA content industrially and thereby mass producing the yeast culture, it is preferable to conduct fed batch cultivation or continuous cultivation.

In addition, there is no particular limitation on the conditions for culturing the *Saccharomyces cerevisiae* mutant strain of the present invention, and the yeast cells can be cultured under the conditions generally employed for culturing the *Saccharomyces* yeasts. For example, the incubation temperature is preferably within a range of 20 to 40°C, and more preferably within a range of 25 to 35°C. In addition, pH of the culture medium is preferably within a range of 3.5 to 8.0, and more preferably within a range of 4.0 to 6.0. In particular, when mass producing the yeast culture industrially, it is preferable to measure the pH of the culture medium on a regular basis and to adjust the pH so as to maintain the pH range from 4.0 to 6.0. Additionally, it is preferable to culture the *Saccharomyces cerevisiae* mutant strain of the present invention under aerobic conditions in a similar manner to that for culturing other *Saccharomyces* yeasts. For example, when growing yeast cells in a batch culture, aeration conditions are preferably within a range of 0.5 to 2 wm, and more preferably within a range of 0.8 to 1.5 vvm.

For example, by culturing the *Saccharomyces cerevisiae* mutant strain of the present invention in a batch culture using the YPD medium under the conditions of an incubation temperature of 25 to 35°C, a rotation speed of 180 to 400 rpm, an aeration of 0.8 to 1.5 wm, and a pH of 4.0 to 5.0, it is possible to achieve the RNA content of 14% by weight or more, based on the dry cell weight, in the *Saccharomyces cerevisiae* mutant strain of the present invention. In addition to these culture conditions, by setting the incubation period within a range of 7 to 12 hours, the *Saccharomyces cerevisiae* mutant strain of the present invention can achieve the RNA content of 18% by weight or more based on the dry cell weight.

By culturing the *Saccharomyces cerevisiae* mutant strain of the present invention, a culture with a sufficiently high RNA content can be obtained, as compared to the case where a known strain of *Saccharomyces cerevisiae* is used. For this reason, by using the obtained culture, it is possible to prepare a yeast extract with a high content of nucleic acids, that is, an RNA content of 35% by weight or more. In particular, it is also possible to prepare a favorable yeast extract in which the total content of inosinic acid (IMP) and guanylic acid (GMP) is 20% by weight or more, and thus the strength of flavor components is increased.

There is no particular limitation on the process for preparing a yeast extract from the culture of the *Saccharomyces cerevisiae* mutant strain of the present invention, and any preparation method which is commonly employed for preparing yeast extracts may be used. Examples of the preparation methods include an autolysis method in which yeast cells are solubilized using an protease or the like which is intrinsic to the yeast cells, an enzymolysis method in which yeast cells are solubilized by adding an enzyme preparation originating from microbes or plants, a hot water extraction method in which yeast cells are solubilized by immersing the cells in a hot water for a given period of time, an acid/alkaline degradation method in which yeast cells are solubilized by adding various acids or alkalis, a freezing and thawing method in which yeast cells are crushed by conducting a freezing and thawing process at least once, and a physical crushing method in which yeast cells are crushed by physical stimuli. Examples of the physical stimuli in the physical crushing methods include an ultrasonic treatment, a high pressure homogenization treatment, and a grinding treatment due to the mixing of solid matter such as glass beads.

Additionally, by subjecting the culture of the *Saccharomyces cerevisiae* mutant strain of the present invention to a drying process, it is possible to obtain dry yeast cells having a high RNA content. There is no particular limitation on the method for subjecting the culture to a drying process, and any preparation method which is commonly employed for preparing dry yeast cells may be used. Examples of the preparation methods include a freeze drying method, a spray drying method, and a drum drying method. Moreover, by processing the obtained dry yeast cells into a powdery form, a dry yeast powder having excellent handling characteristics and a high RNA content can be obtained.

The culture of the *Saccharomyces cerevisiae* mutant strain of the present invention itself, or preparation materials prepared from the culture such as a yeast extract and dry yeast cells can be suitably used, in particular, as a food additive including a flavoring material. There is no particular limitation on the food and beverage products to which the culture or the preparation material is added, and examples thereof include an alcoholic beverage, a soft drink, a fermented food, a flavoring material, soups, bread, and confectionery. In addition, the above culture and the like can also be ingested, as a supplement or the like, when processed into a soft capsule preparation, a hard capsule preparation, a tablet, or the like.

### [Examples]

The present invention will be described below in further detail using Examples. However, the present invention is not limited to the following Examples.

### [Example 1] Production of yeast having a high RNA content

First, the ABYC 1591 strain was inoculated onto an YPD plate medium containing 2% agar and incubated overnight, as a static culture, in an incubator set at 30°C. The subculture plate prepared as a result of the incubation was preserved at 4°C.

One colony of the ABYC 1591 strain was picked from the subculture plate using a platinum loop and inoculated in 50 mL of an YPD liquid medium, and the resultant was incubated overnight at 30°C so as to prepare a preculture liquid. Then 1.5 L of fresh YPD liquid medium was added in a jar fermenter (manufactured by B. E. Marubishi Co., Ltd.) having a volume of 5 L followed by the addition of the preculture liquid thereto so that the inoculation would achieve the initial cell concentration of 1 × 10⁷ cells/ml, and the cells were incubated as a batch culture under the conditions of an incubation temperature of 30°C, a rotation speed of 200 rpm, and an aeration of 1 vvm. The pH of the medium during the incubation of the batch culture was maintained at 4.5 by the use of aqueous ammonia.

9 to 21 hours after the commence of the incubation, a 50 mL aliquot of the culture (YPD liquid medium containing the ABYC 1591 strain) was collected, and the RNA content of the ABYC 1591 strain in the culture was determined. Note that the RNA content in the yeast cells was measured, specifically, by the method described below.

First, by treating the collected yeast culture through a centrifugal separation process, the yeast cells contained therein were recovered as a pellet, and the recovered cells were washed with distilled water. Then the recovered cells were suspended by adding an adequate amount of distilled water so that the absorbance at 660 nm (that is, optical density (OD₆₆₀)) was about 50 to 100, and the resulting suspension was used as a measurement sample. 250 µL of the measurement sample and 250 µL of a 3.3 M sodium chloride solution were mixed, and the resulting mixture was heated at 100°C for 1 hour. After the mixture was cooled down to room temperature, 750 mL of distilled water was added thereto to prepare a blank solution.

Subsequently, to 150 µL of the blank solution, 200 µL of a 0.1 M sodium phosphate buffer (pH 5.3), 50 µL of a 0.01 M zinc chloride solution, and 100 µL of a 39.7 U/ml nuclease P1 solution (manufactured by Yamasa Corporation) were respectively added, and the reaction was conducted at 60°C for 2 hours to degrade the nucleic acids in the blank solution. The obtained solution after the degradation was used as a sample solution.

Both the blank solution and the sample solution were subjected to an ultrafiltration process using an Ultrafree (registered trade mark) -MC Centrifugal Filter Units (10,000 NMWL, manufactured by Millipore Corporation), and the content of nucleic acids in each of the solution was measured using a high performance liquid chromatograph (HPLC). The nucleic acid component was calculated as disodium salt heptahydrate. Note that the measurement using the HPLC was conducted under the following conditions.
HPLC apparatus: LC-10ADVP (manufactured by Shimadzu Corporation);
Column: HitachiGel #3013-N packed column;
Detector: SPD-10AVP (manufactured by Shimadzu Corporation);
Mobile phase: 67 mM NH₄Cl, 10 mM KH₂PO₄, 20 mM K₂HPO₄, 6% acetonitrile solution;
Injection: 10 µL;
Column temperature: 35°C;
Flow rate: 1.0 mL/min;
Detection wavelength: 254 nm;
Analysis time: 30 minutes.

**[Table 3]**

| Time point after commencing of incubation | 9 hours | 15 hours | 18 hours | 21 hours |
|---|---|---|---|---|
| RNA (% by weight) | 21.4 | 20.4 | 16.7 | 15.0 |

Table 3 is a table showing the time points and the RNA content of yeast cells in the culture at each time points after the commencing of the incubation. From these results it was apparent that the RNA content of yeast cells in the culture was highest at the time point of 9 hours after the commencing of the incubation, and the RNA content was 21.4% by weight. In addition, a considerably high RNA content of 16.3% by weight was also observed at the time point of 18 hours after the commencing of the incubation. As shown in Comparative Example 1 described later, a known strain of *Saccharomyces cerevisiae* usually has an RNA content of 10% by weight or less at the time point of 18 hours after the commence of the incubation. In other words, from the results shown in Example 1, it is apparent that the *Saccharomyces cerevisiae* mutant strain of the present invention is a yeast strain having a considerably high RNA content.

### [Example 2] Preparation of yeast extract

First, the ABYC 1591 strain was inoculated in the YPD medium and cultured, in a similar manner to that described in Example 1, and the culture was obtained at the time point of 18 hours after the commence of the incubation. When the RNA content of the ABYC 1591 strain in the obtained culture was measured in a similar manner to that described in Example 1, it was 14.6% by weight based on the dry cell weight.

Then, by treating the obtained yeast culture through a centrifugal separation process, the yeast cells contained therein were recovered as a pellet, and the recovered cells were washed with distilled water. Thereafter, a yeast cell suspension was prepared by adding an adequate amount of distilled water so as to achieve a cell concentration of 10 to 15%. After a sterilization process, the yeast cell suspension was adjusted so as to achieve a pH of 5, and a cell wall digesting enzyme was added thereto followed by the incubation at 50°C for 17 hours. Then a supernatant extract was recovered by treating the obtained yeast suspension through a centrifugal separation process, and after the pH adjustment so as to achieve a pH of 5.5, a ribonuclease and a deaminase were added to the resulting supernatant extract and an enzymatic reaction was carried out at 50°C for 17 hours. After inactivating the enzymes by heating the mixture of supernatant extract to 80°C, the mixture was filter sterilized and then dried to prepare a yeast extract.

When the RNA content in the obtained yeast extract was measured in a similar manner to that described in Example 1, it was 43.4% by weight. In addition, the total content of inosinic acid and guanylic acid, which were taste components, was 24.4% by weight.

From these results, it is apparent that by using the *Saccharomyces cerevisiae* mutant strain of the present invention, it is possible to prepare a favorable yeast extract having a high RNA content.

### [Comparative Example 1] Preparation of yeast extract

Apart from the use of a *Saccharomyces cerevisiae* S288C strain instead of the ABYC 1591 strain, a yeast culture was obtained in a similar manner to that described in Example 1. When the RNA content of the S288C strain in the obtained culture was measured in a similar manner to that described in Example 1, it was 8.5% by weight based on the dry cell weight.

Then, a yeast extract was prepared from the obtained culture in a similar manner to that described in Example 2, and the RNA content in the yeast extract was measured. The results showed that the RNA content in the yeast extract prepared from the culture of the S288C strain was 17.2% by weight, and the total content of inosinic acid and guanylic acid, which were taste components, was 7.9% by weight.

In addition, a sensory evaluation was conducted on the yeast extract prepared in Example 2 and on the yeast extract prepared in Comparative Example 1.

Specifically, both yeast extracts were dissolved, respectively, in a warm water of 40°C so as to achieve a concentration of 1%, as a result of which yeast extract solutions were prepared. When these yeast extract solutions were tasted, the yeast extract solution of Example 2 was richer in taste and had a deeper flavor, as compared to the yeast extract solution of Comparative Example 1.

In addition, both yeast extracts were added, respectively, to commercially available consomme soup so as to achieve a concentration of 1%. As a result, the soup to which the yeast extract of Example 2 was added had a deeper flavor and thus favorable taste, as compared to the soup to which the yeast extract of Comparative Example 1 was added.

These results are considered to be due to the fact that the yeast extract of Example 2 contains twice as much as the yeast extract of Comparative Example 1 does or even more, in terms of the RNA content and the total content of inosinic acid and guanylic acid.

### INDUSTRIAL APPLICABILITY

Since the *Saccharomyces cerevisiae* mutant strain of the present invention has a high RNA content and it is possible to simply and easily produce a yeast culture or a yeast extract having a high RNA content by means of culturing the mutant strain, the present invention can be used, in particular, in the field of food industry and the like.

## Claims

1. The *Saccharomyces cerevisiae* ABYC 1591 strain, deposited under accession number FERM BP-10925, at the International Patent Organism Depositary on October 19, 2007.

2. A method for producing a yeast of high RNA content, the method comprising:
a step of culturing the *Saccharomyces cerevisiae* strain according to claim 1, and thereby making the strain accumulate, in the cells thereof, 14% by weight or more of RNA based on the dry cell weight.

3. A culture of the *Saccharomyces cerevisiae* strain according to claim 1.

4. The culture according to claim 3, wherein the RNA content is 14% by weight or more based on the dry cell weight.

5. The culture according to claim 4, wherein the RNA content is 18% by weight or more based on the dry cell weight.

6. A yeast extract prepared from a culture of the *Saccharomyces cerevisiae* mutant strain according to claim 1.

7. The yeast extract according to claim 6, wherein a RNA content is 35% by weight or more.

8. The yeast extract according to claim 7, wherein the total content of inosinic acid (IMP) and guanylic acid (GMP) is 20% by weight or more.

## Patentansprüche

1. *Saccharomyces cerevisiae* ABYC 1591 Stamm, hinterlegt bei der Internationalen Patent Organismus Hinterlegungsstelle (International Patent Organism Depositary) am 19. Oktober 2007 unter der Hinterlegungsnummer FERM BP-10925.

2. Verfahren zum Herstellen einer Hefe von hohem RNA-Gehalt, wobei das Verfahren umfasst:
einen Schritt des Kultivierens des *Saccharomyces cerevisiae*-Stamms gemäß Anspruch 1, und dadurch bewirken des Stamms in den Zellen davon 14 Gew.-% oder mehr von RNA basierend auf dem Trockenzellgewicht anzuhäufen.

3. Kultur des *Saccharomyces cerevisiae*-Stamms gemäß Anspruch 1.

4. Kultur nach Anspruch 3, wobei der RNA-Gehalt 14 Gew.-% oder mehr basierend auf dem Trockenzellgewicht beträgt.

5. Kultur nach Anspruch 4, wobei der RNA-Gehalt 18 Gew.-% oder mehr basierend auf dem Trockenzellgewicht beträgt.

6. Hefeextrakt, hergestellt aus einer Kultur des *Saccharomyces cerevisiae* Mutantenstamms gemäß Anspruch 1.

7. Hefeextrakt nach Anspruch 6, wobei ein RNA-Gehalt 35 Gew.-% oder mehr beträgt.

8. Hefeextrakt nach Anspruch 7, wobei der Gesamtgehalt an Inosinsäure (IMP) und Guanylsäure (GMP) 20 Gew.-% oder mehr beträgt.

## Revendications

1. Souche de *Saccharomyces cerevisiae* ABYC 1591, déposée sous le numéro d'accès FERM BP-10925, à l'International Patent Organism Depositary le 19 octobre 2007.

2. Procédé de production d'une levure à haute teneur en ARN, le procédé comprenant :
une étape de culture de la souche de *Saccharomyces cerevisiae* selon la revendication 1, et faisant ainsi accumuler par la souche, dans les cellules de celle-ci, 14 % en poids ou plus d'ARN sur la base du poids de cellules sèches.

3. Culture de la souche de *Saccharomyces cerevisiae* de la revendication 1.

4. Culture selon la revendication 3, dans laquelle la teneur en ARN est de 14 % en poids ou plus sur la base du poids de cellules sèches.

5. Culture selon la revendication 4, dans laquelle la teneur en ARN est de 18 % en poids ou plus sur la base du poids de cellules sèches.

6. Extrait de levure préparé à partir d'une culture de la souche mutante de *Saccharomyces cerevisiae* de la revendication 1.

7. Extrait de levure selon la revendication 6, dans lequel une teneur en ARN est de 35 % en poids ou plus.

8. Extrait de levure selon la revendication 7, dans lequel la teneur totale en acide inosinique (IMP) et acide guanylique (GMP) est de 20 % en poids ou plus.
